# EUROPÄISCHE PATENTSCHRIFT

(11) **EP 3 668 489 B1**
(45) Veröffentlichungstag und Bekanntmachung des Hinweises auf die Patenterteilung: **15.09.2021**
(21) Anmeldenummer: 18756408.3
(22) Anmeldetag: 13.08.2018
(51) Int. Cl.: A61K 9/107, A61K 47/42, A61K 31/07, A23L 33/19, A61K 31/122, A61K 31/355, A61K 31/592, A61K 31/593

(54) **WASSERMISCHBARES PRÄPARAT EINER LIPOPHILEN VERBINDUNG**
WATER-MISCIBLE PREPARATION OF A LIPOPHILIC COMPOUND
PRÉPARATION MISCIBLE À L'EAU D'UN COMPOSÉ LIPOPHILE

(30) Priorität: 16.08.2017 DE 102017214305
(43) Veröffentlichungstag der Anmeldung: 24.06.2020
(73) Patentinhaber: Deutsches Institut für Lebensmitteltechnik e.V., 49610 Quakenbrück (DE)
(72) Erfinder: BINDRICH, Ute, 49610 Quakenbrück (DE)
(74) Vertreter: Taruttis, Stefan Georg
(86) Internationale Anmeldenummer: PCT/EP2018/071956
(87) Internationale Veröffentlichungsnummer: WO 2019/034624

(56) Entgegenhaltungen:
- HENRIKE MOELLER ET AL: "Native casein micelles as nanocarriers for [beta]-carotene: pH-and temperature-induced opening of the micellar structure", INTERNATIONAL JOURNAL OF FOOD SCIENCE AND TECHNOLOGY., Bd. 52, Nr. 5, 3. April 2017 (2017-04-03), Seiten 1122-1130, XP055515876, GB ISSN: 0950-5423, DOI: 10.1111/ijfs.13387
- TAVARES GUILHERME M ET AL: "Milk proteins as encapsulation devices and delivery vehicles: Applications and trends", TRENDS IN FOOD SCIENCE AND TECHNOLOGY, Bd. 37, Nr. 1, 31. Dezember 2014 (2014-12-31), Seiten 5-20, XP028655072, ISSN: 0924-2244, DOI: 10.1016/J.TIFS.2014.02.008
- SHARMA PANKAJ ET AL: "Effect of pulsed electric field processing on the functional properties of bovine milk", TRENDS IN FOOD SCIENCE AND TECHNOLOGY, Bd. 35, Nr. 2, 31. Dezember 2014 (2014-12-31), Seiten 87-101, XP028608249, ISSN: 0924-2244, DOI: 10.1016/J.TIFS.2013.11.004
- BUCKOW ROMAN ET AL: "Opportunities and challenges in pulsed electric field processing of dairy products", INTERNATIONAL DAIRY JOURNAL, Bd. 34, Nr. 2, 31. Dezember 2013 (2013-12-31), Seiten 199-212, XP028774767, ISSN: 0958-6946, DOI: 10.1016/J.IDAIRYJ.2013.09.002

## Beschreibung

Die vorliegende Erfindung betrifft ein Verfahren zur Herstellung eines mit Wasser oder hydrophilen Lebensmitteln mischbaren Präparats zumindest einer lipophilen Verbindung, die z.B. ein lipophiles Vitamin, Provitamin, oder eine Mischung aus zumindest zweien dieser sein kann. Daher betrifft die Erfindung auch die Herstellung von Lebensmitteln, die das Präparat enthalten. Solche Lebensmittel und Nahrungsergänzungsmittel können fettreduziert bzw. fettfrei sein, aus einer hydrophilen Phase bestehen und/oder keine Emulsion sein und bevorzugt keinen Emulgator enthalten. Das Präparat hat den Vorteil, dass die darin enthaltene lipophile Verbindung mit Wasser oder hydrophilen Lebensmitteln bzw. Nahrungsergänzungsmitteln mischbar ist, aus natürlichen Bestandteilen besteht, kein zugesetztes Fett und keinen Emulgator enthält, und bevorzugt fettarm, z.B. maximal 2 Gew.-% Fett enthält, oder fettfrei ist. Die lipophile Verbindung ist bevorzugt in dem Präparat stabilisiert, z.B. gegen UV-Strahlung und/oder gegen Oxidation. Entsprechend kann die lipophile Verbindung selbst z.B. gegenüber UV-Strahlung und/oder Sauerstoffinstabil sein und ist in dem Präparat gegen UV und/oder Oxidation, insbesondere gegen Sauerstoff, stabilisiert.

### Stand der Technik

Moeller et al., International Jorunal of Food Science and Technology, 52, 1122-1130 (2017) beschreibt, dass native Caseinmicellen als Träger zum Einschluss von z.B. fettlöslichen Mikronährstoffen wie Beta-Carotin geeignet sind. Caseinmicellen wurden durch Kühlung auf 2°C in Kombination mit einer Absenkung des pH-Werts auf 5,5 mit Beta-Carotin beladen.

Tavares et al., Trends in Food Science & Technology 37, 5-20 (2014) beschreibt die Verkapselung von lipophilen Substanzen in Caseinmicellen - ähnlichen Strukturen, die aus Caseinat erzeugt werden konnten, durch Reduktion des pH-Werts, z.B. durch Carbonisierung, und den Einsatz von Labenzym, oder durch Erhöhung der Ionenkonzentration und Absenkung des pH-Werts, oder aus Caseinat und Tri-Kalium-Phosphat durch Zusatz von Kalium-Hydrogen-Phosphat und Calciumchlorid.

Buckow et al., International Dairy Journal 34, 199-212 (2014) beschreibt, das gepulste elektrische Felder (PEF) zum Haltbarmachen von Milch eingesetzt werden können.

Sharma et al., Trends in Food Science & Technology 35, 87-101 (2014) beschreibt die Wirkung von gepulsten elektrischen Feldern auf Kuhmilch, sowie den Einfluss dieser Behandlung auf den Gehalt verschiedener Vitamine.

### Aufgabe der Erfindung

Die Aufgabe der Erfindung liegt darin, ein Verfahren zur Herstellung eines Präparats bereitzustellen, das mit Wasser und hydrophilen Lebensmitteln, insbesondere fettfreien Lebensmitteln, mischbar ist und eine lipophile Verbindung enthält.

### Beschreibung der Erfindung

Die Erfindung löst die Aufgabe mit den Merkmalen der Ansprüche, insbesondere mit Verfahren, bei dem Caseinmicellen, die in einem wässrigen Medium suspendiert sind, mit einem ersten gepulsten elektrischen Feld behandelt werden, dann die erhaltene Suspension erster Caseinmicellen mit zumindest einer lipophilen Verbindung gemischt werden, und anschließend diese Mischung mit einem zweiten gepulsten elektrischen Feld behandelt wird, um Caseinmicellen, die die zumindest eine lipophile Verbindung enthalten, als Präparat herzustellen, das in dem wässrigen Medium suspendiert ist. Das Präparat zeichnet sich dadurch aus, dass es Caseinmicellen und zumindest eine lipophile Verbindung aufweist oder daraus besteht, wobei die lipophile Verbindung mit den Caseinmicellen verbunden ist, bevorzugt von den Caseinmicellen umfasst ist. Die Caseinmicellen sind aus Milch erhältlich, z.B. durch Mikrofiltration aus Magermilch, optional aus dem getrockneten Zustand rehydratisiert.

Das Präparat enthält die lipophile Verbindung, die optional gegenüber UV und/oder Sauerstoff instabil sein kann, in stabilisierter Form. Gegenwärtig wird angenommen, dass die lipophile Verbindung von den Caseinmicellen umfasst bzw. eingeschlossen ist und so gegen UV und/oder Oxidation, z.B. durch Sauerstoff, geschützt wird.

Die Freisetzung der lipophilen Verbindung aus dem Präparat erfolgt durch die normale Verdauung, so dass die lipophile Verbindung nach der Nahrungsaufnahme aus dem Präparat freigesetzt wird.

Das Präparat ist durch ein Verfahren erhältlich, das die folgenden Schritte aufweist oder daraus besteht:
- Behandeln einer Suspension von Caseinmicellen in wässrigem Medium mit einem ersten gepulsten elektrischen Feld (PEF-1) mit einem spezifischen Energieeintrag von 20 bis 150 kJ/kg, bevorzugt 35 kJ/kg der wässrigen Suspension, einer elektrischen Feldstärke von 10 bis 20 kV/cm, bevorzugt 11,5 kV/cm, und einer Pulsdauer von 15 bis 25 µs, bevorzugt 15 µs, bevorzugt bei einer Pulsfrequenz von 150 bis 250 Hz, wobei die wässrige Suspension bevorzugt vor der Behandlung mit dem ersten gepulsten elektrischen Feld eine Temperatur von 1 bis 60 °C aufweist, zur Herstellung einer Suspension erster Caseinmicellen,
- Mischen der Suspension erster Caseinmicellen mit einer Lösung der lipophilen Verbindung, deren Lösungsmittel bevorzugt mit dem wässrigen Medium mischbar ist, zur Herstellung einer Mischung der Suspension erster Caseinmicellen mit der lipophilen Verbindung, wobei bevorzugt diese Mischung für z.B. 1 bis 15 min gemischt wird,
- Behandeln der Mischung der Suspension erster Caseinmicellen mit der lipophilen Verbindung, die bevorzugt eine Temperatur von 35 bis 60 °C aufweist, mit einem zweiten gepulsten elektrischen Feld (PEF-2) mit einem spezifischen Energieeintrag von 40 bis 61 kJ/kg der Mischung, z.B. bei einer elektrischen Feldstärke von 10 bis 20 kV/cm, bevorzugt 11,5 kV/cm, und einer Pulsdauer von 15 bis 25 µs, bevorzugt 15 µs, bevorzugt bei einer Pulsfrequenz von 150 bis 250 Hz, zur Herstellung von Caseinmicellen,
- optional mit einem anschließenden Heißhalten, z.B. bei 55 bis 65 °C, bevorzugt 60 °C, für z.B. 5 bis 10 min, optional mit oder ohne Bewegung der Mischung, zur Herstellung einer Suspension des Präparats mit oder aus Caseinmicellen, die die lipophile Verbindung enthalten,
- bevorzugt ohne Zusetzen eines Emulgators oder Tensids
- optional Abtrennen des Präparats von der flüssigen Phase und/oder
- optional Trocknen des Präparats zur Herstellung des getrockneten Präparats.

Die Behandlung mit dem ersten elektrischen Feld und/oder die Behandlung mit dem zweiten elektrischen Feld erfolgt bevorzugt im Durchfluss, z.B. bei einer Durchflussgeschwindigkeit von 20 bis 30 L/h. Die Behandlung mit dem ersten elektrischen Feld und/oder die Behandlung mit dem zweiten elektrischen Feld kann jeweils unabhängig voneinander mit gleichbleibender oder alternierender Polarität zwischen zwei Elektroden erfolgen, bevorzugt mit jeweils alternierender Polarität.

Für native Caseinmicellen, die z.B. durch Mikrofiltration aus Milch, bevorzugt aus fettarmer Milch, abgetrennt wurden, wird die Mischung der Suspension erster Caseinmicellen mit der lipophilen Verbindung vor der Behandlung mit dem zweiten gepulsten elektrischen Feld bevorzugt auf 35 bis 45 °C temperiert, der spezifische Energieeintrag der PEF-2 beträgt bevorzugt 40 bis 55 kJ/kg, und das anschließende Heißhalten ist bevorzugt, z.B. für 60 °C für 5 bis 10 min. Für Caseinmicellen, die in dem Medium rehydratisiert vorliegen, z.B. rehydratisierte, zuvor getrocknete oder gefriergetrocknete Caseinmicellen, wird die Mischung der Suspension erster Caseinmicellen mit der zumindest einen lipophilen Verbindung vor der Behandlung mit dem zweiten gepulsten elektrischen Feld bevorzugt auf eine Temperatur von 50 bis 60 °C temperiert, bevorzugt beträgt der Energieeintrag der PEF-2 51 bis 61 kJ/kg und bevorzugt erfolgt anschließend an PEF-2 ein Heißhalten bei 60 °C für 5 bis 10 min.

Die Mischung der Suspension erster Caseinmicellen mit der lipophilen Verbindung wird vor der Behandlung mit dem zweiten gepulsten elektrischen Feld bevorzugt auf 50 bis 60 °C temperiert, der spezifische Energieeintrag der PEF-2 beträgt bevorzugt 51 bis 61 kJ/kg, und das anschließende Heiß halten ist bevorzugt, z.B. für 60 °C für 5 bis 10 min.

Das wässrige Medium mit den darin suspendierten Caseinmicellen weist bevorzugt die Caseinmicellen in einem Proteingehalt von 2 bis 10 g /100 g Suspension, vorzugsweise 3,0 g / 100 g Suspension auf, bevorzugt einen pH-Wert von 6,2 bis 6,4, bevorzugt pH 6,3, bevorzugt eine Leitfähigkeit von 0,8 bis 1,5 mS/cm, bevorzugter von 1,2 mS/cm, und bevorzugt eine Temperatur von 2,5 bis 3,5 °C, bevorzugter 3,0 °C.

Das Präparat, das die Caseinmicellen, die die lipophile Verbindung enthalten, aufweist oder daraus besteht, kann als wässrige Suspension oder als getrocknetes Präparat in Lebensmittelmassen eingemischt werden, die insofern fettarm oder fettfrei sind, dass sie kein freies Fett enthalten, in das die lipophile Verbindung alleine nicht gelöst werden kann.

Das Präparat hat den Vorteil, die zumindest eine lipophile Verbindung in einer mit wässrigen Lebensmittelmassen mischbaren Form zu enthalten. Bevorzugt enthält das Präparat die lipophile Verbindung stabil, so dass diese auch nach Einmischen in eine Lebensmittelmasse im Wesentlichen mit den Caseinmicellen verbunden bleibt, bevorzugt in den Caseinmicellen eingeschlossen bleibt.

Die lipophile Verbindung kann ein fettlösliches Vitamin sein, z.B. Vitamin A, D, E und/oder K oder ein Provitamin, z.B. β-Carotin oder eine Mischung von zumindest zweien dieser sein.

Die Lebensmittelmasse kann z.B. ein Milchprodukt sein, z.B. Milch, Joghurt, Kefir, Quark, Frischkäse, Käse, Buttermilch, Pudding, milchhaltiges oder milchfreies Speiseeis, oder Süßwarenmassen, z.B. Schokolade, Geleemasse, Kaugummimasse, oder Wurstwaren und vegetarische Lebensmittel mit geringen Fettgehalten.

Das Trocknen des Präparats kann durch Gefriertrocken erfolgen, bevorzugt durch Wirbelschichttrocken, z.B. bei maximal 80 °C, bevorzugter maximal 70 oder maximal 60 °C.

Bevorzugt wird das Präparat bei einer Temperatur in eine Lebensmittelmasse eingemischt, bei der die lipophile Verbindung in dem Präparat im Wesentlichen erhalten bleibt. Das Präparat kann in gleicher Weise bei der Lebensmittelherstellung eingesetzt werden wie übliche caseinreiche Milchproteinkonzentrate oder getrocknete caseinreiche Milchproteinpulver.

Die Erfindung wird nun anhand von Beispielen mit Bezug auf die Figuren beschrieben, die in
- Figur 1 den Gehalt eines Präparats mit nativen Caseinmicellen an lipophiler Verbindung in Abhängigkeit von Verfahrensbedingungen und dem spezifischen Energieeintrag der PEF-2 (PEF2),
- Figur 2 die spezifische Oberfläche der Caseinpartikel des Präparats mit nativen Caseinmicellen in Abhängigkeit von der Temperatur der Mischung vor PEF-2 und dem spezifischen Energieeintrag der PEF-2,
- Figur 3 das Oberflächenpotential der Caseinpartikel des Präparats mit nativen Caseinmicellen in Abhängigkeit von der Temperatur der Mischung vor PEF-2 und dem spezifischen Energieeintrag der PEF-2,
- Figur 4 die Ladungsdichte der Caseinpartikel des Präparats mit nativen Caseinmicellen in Abhängigkeit von der Temperatur der Mischung vor PEF-2 und dem spezifischen Energieeintrag der PEF-2,
- Figur 5 den Gehalt eines Präparats mit rehydratisierten Caseinmicellen an lipophiler Verbindung in einem Präparat in Abhängigkeit von Verfahrensbedingungen der PEF-2,
- Figur 6 den Gehalt des Präparats mit rehydratisierten Caseinmicellen an lipophiler Verbindung in einem Präparat in Abhängigkeit von Verfahrensbedingungen der PEF-2 in Bezug zu einem thermischen Stabilisierungsprozess (Vergleich, nur Erhitzung für 30 min bei 60°C) in %,
- Figur 7 die spezifische Oberfläche der Caseinpartikel des Präparats mit rehydratisierten Caseinmicellen in Abhängigkeit von der Temperatur der Mischung vor PEF-2 und dem spezifischen Energieeintrag der PEF-2
- Figur 8 das Oberflächenpotential der Caseinpartikel des Präparats mit rehydratisierten Caseinmicellen in Abhängigkeit von der Temperatur der Mischung vor PEF-2 und dem spezifischen Energieeintrag der PEF-2 und in
- Figur 9 die Ladungsdichte der Caseinpartikel des Präparats mit rehydratisierten Caseinmicellen in Abhängigkeit von der Temperatur der Mischung vor PEF-2 und dem spezifischen Energieeintrag der PEF-2.

### Beispiel 1: Präparat aus nativen Caseinmicellen mit β-Carotin

Native Caseinmicellen wurden durch Mikrofiltration aus Magermilch gewonnen und in Molke auf eine Proteinkonzentration der Caseinmicellen von 3,0 g / 100 g eingestellt, der pH wurde auf 6,3 eingestellt und die Leitfähigkeit durch Zugabe von NaCl auf 1,2 mS/cm. Diese Suspension wurde auf 3,0 °C temperiert und kontinuierlich bei einer Durchflussrate von ca. 25 L/h in einer Behandlungszelle, in der zwei kolineare Wandungsabschnitte aus Titan als Elektroden ausgebildet waren und durch einen Isolator aus Aluminiumoxid beabstandet waren, mit ersten gepulsten elektrischen Feldern alternierender Polarität beaufschlagt. Der spezifische Energieeintrag war 35 kJ/kg Suspension, die elektrische Feldstärke betrug 11,5 W/cm, die Pulsfrequenz 200 Hz bei einer Pulsdauer von 15 ms. Als Beispiel für eine lipophile Verbindung wurde β-Carotin in Ethanol als Lösungsmittel zu 0,1 g /L durch 15 min Rühren und 30 s Ultraschall gelöst. Die so hergestellte Suspension erster Caseinmicellen wurde je Liter mit 60 mL einer alkoholischen 1,2 mg/ 100 g β-Carotinlösung für ca. 10 min gemischt. Wegen der Lichtempfindlichkeit von β-Carotin wurde diese Lösung und die anschließende Mischung mit der ersten Suspension von Caseinmicellen im Dunklen gehalten.

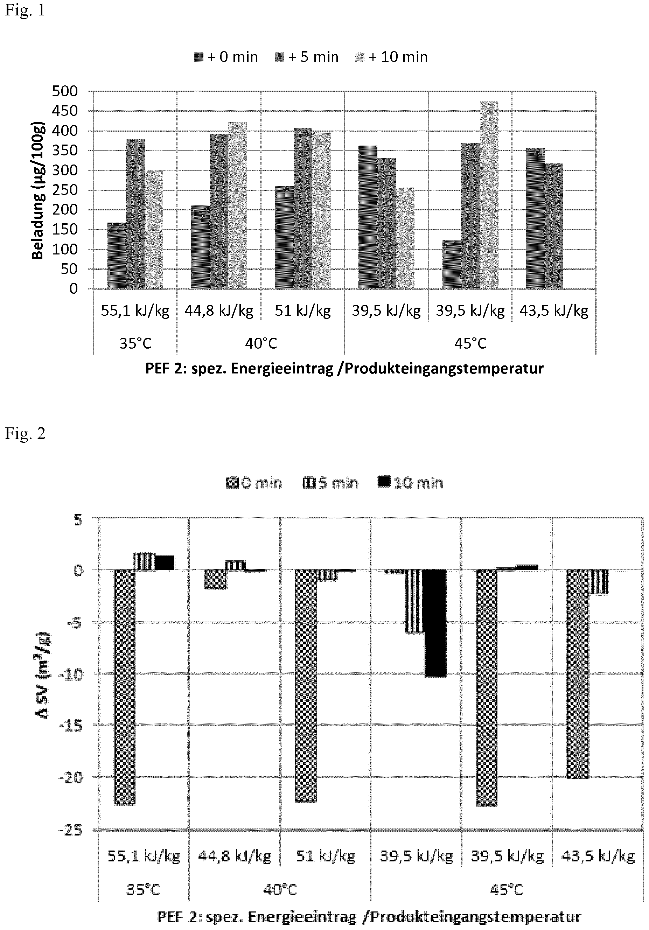

Die so hergestellte Mischung der Suspension erster Caseinmicellen mit der lipophilen Verbindung wurde auf eine Temperatur zwischen 35 und 45 °C temperiert und mit zweiten gepulsten elektrischen Feldern alternierender Polarität beaufschlagt. Der spezifische Energieeintrag wurde für verschiedene Aliquots variiert. Das erhaltene suspendierte Präparat wurde anschließend bei verschiedenen Temperaturen heißgehalten. Der spezifische Energieeintrag (spez. Energieeintrag) dieser PEF-2, die Temperatur der Mischung vor der Beaufschlagung (Produkteingangstemperatur) mit dem zweiten gepulsten elektrischen Feld (PEF-2) und die Temperaturen und Dauer (+ 0 min, + 5 min, + 10 min) der anschließenden Heißhaltung sind in Figuren 1 bis 4 angegeben, die von links nach rechts die Messwerte für dieselben Proben zeigen, die jeweils unter denselben Verfahrensbedingungen hergestellt wurden.

Der Gehalt des Präparats an β-Carotin wurde durch photometrisch nach Auflösung der Caseinmicellen gemessen.

Figur 1 zeigt die jeweils gemessenen Gehalte an β-Carotin bei den angegebenen Werten des verwendeten spezifischen Energieeintrags der PEF-2, der Temperatur der Mischung aus der Suspension erster Caseinmicellen (Produkteingangstemperatur) vor PEF-2 nach verschieden langen und verschieden temperierten Heißhaltungen. Die Ergebnisse zeigen hohe Beladungen der Caseinmicellen mit der lipophilen Verbindung, für β-Casein ca. 125 bis 475 µg/100 g Präparat. Es wird deutlich, dass die Heißhaltung bei einigen Kombinationen von spezifischem Energieeintrag und Produkteingangstemperatur zu einer höheren Beladung der Caseinmicellen mit β-Carotin führte. Eine Ausnahme von der günstigen Wirkung der Heißhaltung waren die Probe, die bei der PEF-2 einen pH von 6,1 aufwies und die Probe mit der höchsten Energieeinwirkung bei PEF-2, Produkteingangstemperatur 45 °C und 43,5 kJ/kg. Bei diesen Proben war die höchste Beladung der Caseinmicellen bereits durch die PEF-2 erreicht.

Die Partikeloberfläche (SV) wurde durch Laserbeugungsspektroskopie (Malvern Mastersizer 2000) entsprechend ISO 13320-1 gemessen und beschreibt die Desintegration bzw. Reassoziierung von Micellen und deren Agglomeraten bzw. ermittelt die spezifische Oberfläche SV der Micellen, über die ein Stofftransport stattfinden kann. Die Ergebnisse sind in Figur 2 in Bezug zu den dem nach PEF-1 für die Suspension erster Caseinmicellen gemessenen Wert dargestellt und zeigen große Änderungen unter den angegebenen Verfahrensbedingungen. Generell zeigt sich, dass die spezifische Partikeloberfläche des Präparats nach der PEF-2 verringert war und mit Ausnahme einer Probe durch die an die PEF-2 anschließende Heißhaltung erhöht wurde. Dies wird darauf zurückgeführt, dass die PEF-2 zu einer Agglomeration der Caseinmicellen führt, die sich in einer Verringerung der spezifischen Oberfläche zeigt. Diese Agglomeration wurde durch die anschließende Heißhaltung spontan bzw. ohne mechanische Belastung des Präparats aufgelöst, so dass mit Ausnahme der Probe bei pH 6,1 im Wesentlichen die spezifische Partikeloberfläche bzw. die Partikelgrößenverteilung durch die Heißhaltung erreicht wurde, die die ersten Caseinmicellen mit der lipophilen Verbindung in der Suspension aufwiesen.

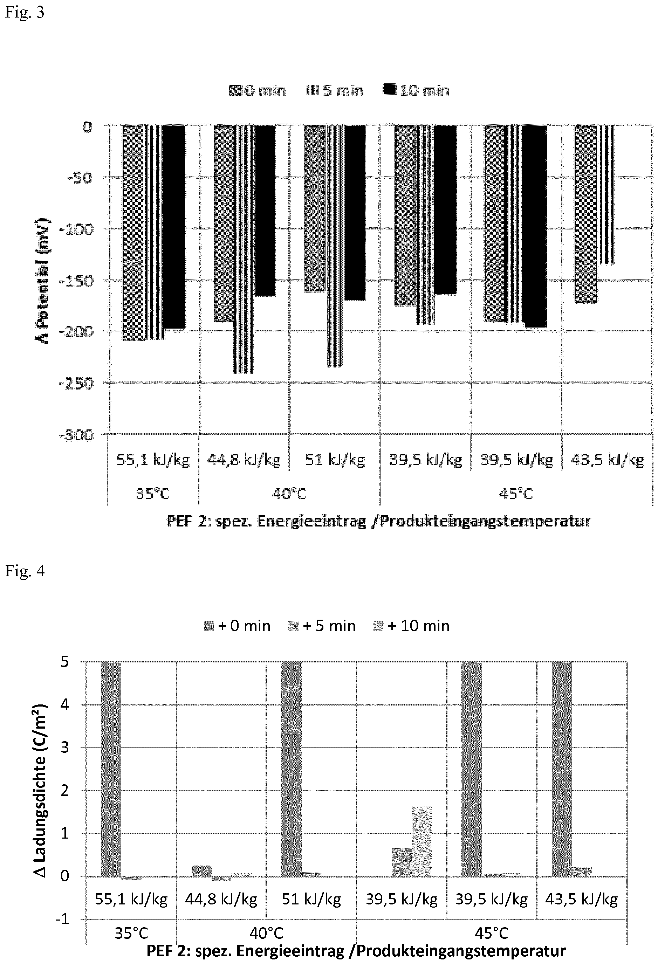

Die Potentiale des Präparats nach der PEF-2 wurde durch Ladungsmessung ermittelt. Die Ergebnisse sind in Figur 3 in Bezug zu den dem nach PEF-1 für die Suspension erster Caseinmicellen gemessenen Wert dargestellt und zeigen, dass das Präparat nach der PEF-2 und auch nach der optionalen zusätzlichen Heißhaltung starke negative Potentiale aufweist. Diese negativen Potentiale werden als erforderlich für die Stabilität des Präparats gegen Agglomeration angesehen bzw. zeigen die Stabilität des Präparats gegen Agglomeration.

Die Oberflächenladung und die Ladungsdichte des Präparats wurden mit einem Ladungsmessgerät CAD (erhältlich von der emtec Electronic GmbH, Leipzig) gemessen. Während der Messung wurde eine Suspension der Caseinmicellen, die mit 0,001 N Polynatriumdiallyldimethylammoniumchlorid (Na-Poly-DADMAC) als Titrationshilfsmittel versetzt war, an einer Messzelle vorbeibewegt. Durch die Bewegung der Suspension werden die Gegenionen, die mit den Caseinmicellen assoziiert sind, in eine ungleichmäßige Verteilung verzerrt, die als positive Ladung bei Kationen oder als negative Ladung bei Anionen oder Nullladung bei keiner Ladung gemessen werden kann. Die Messzelle war eingerichtet, die durch die Bewegung verursachte Ladungsadsorption zu bestimmen. Die Ladungsdichte kann mittels des Verbrauchs an Titrationshilfsmittel in Bezug zur spezifischen Oberfläche der Caseinmicellen bestimmt werden.

Die Ergebnisse sind in Figur 4 in Bezug zu den dem nach PEF-1 für die Suspension erster Caseinmicellen gemessenen Wert dargestellt und zeigen, dass das Präparat nach der PEF-2 hohe Ladungsdichten aufweist, die durch die anschließende Heißhaltung verringert werden, wieder mit der Ausnahme des Präparats bei pH 6,1. Da die Bildung von lockeren Agglomeraten aufgrund der starken Verringerung der Partikeloberfläche mit einer höheren Ladungsdichte verbunden ist, trägt die hohe Ladungsdichte auch zu Auflösung der Agglomerate bei. Darüber hinaus scheint die zusätzliche Heißhaltung im Vergleich mit der Ladungsdichte der Suspension erster Caseinmicellen, die nach PEF-1 bestimmt wurde, keine wesentliche Veränderung der Ladungsdichte zu bewirken.

### Beispiel 2: Präparat aus rehydratisierten Caseinmicellen mit β-Carotin

Das Verfahren zur Herstellung von Beispiel 1 wurde mit Caseinmicellen (erhältlich von Sachsenmilch Ingredients), die aus dem trockenen Zustand in dest. Wasser rehydratisiert wurden, wiederholt.

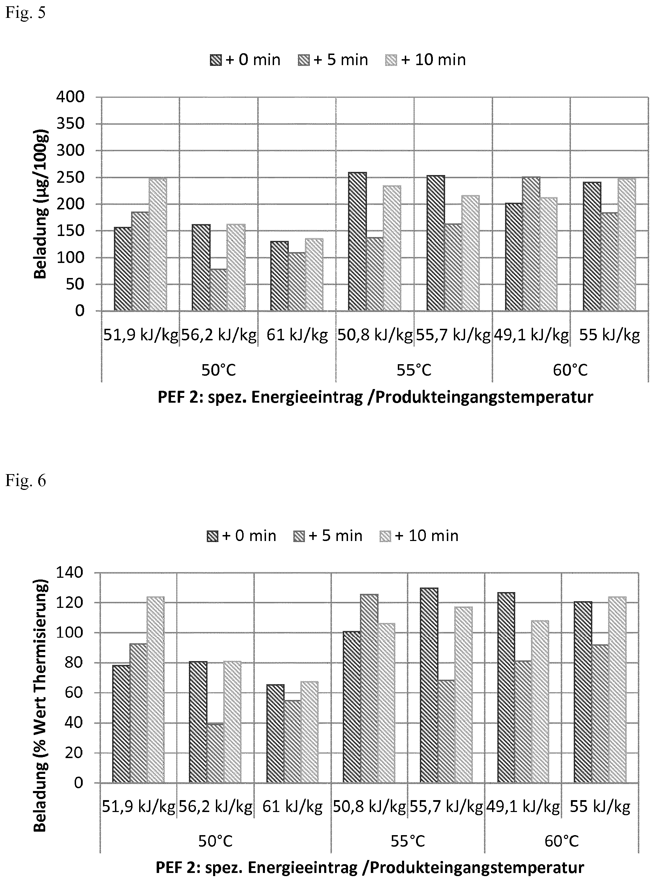

Die Mischung der Suspension erster Caseinmicellen mit β-Carotin als lipophiler Verbindung wurde auf eine Temperatur zwischen 50 und 60 °C temperiert und mit zweiten gepulsten elektrischen Feldern alternierender Polarität beaufschlagt. Der spezifische Energieeintrag wurde für verschiedene Aliquots variiert. Das erhaltene suspendierte Präparat wurde anschließend bei verschiedenen Temperaturen heißgehalten. Der spezifische Energieeintrag (spez. Energieeintrag) dieser PEF-2, die Temperatur der Mischung vor der Beaufschlagung (Produkteingangstemperatur) mit dem zweiten gepulsten elektrischen Feld (PEF-2) und die Temperaturen und Dauer (+ 0 min, + 5 min, + 10 min) der anschließenden Heißhaltung sind in Figuren 5 bis 9 angegeben, die von links nach rechts (Säulen in Figuren) die Messwerte für dieselben Proben zeigen, die jeweils unter denselben Verfahrensbedingungen hergestellt wurden.

Die Messwerte für den Gehalt des Präparats an β-Carotin sind in Figur 5 absolut und Figur 6 in % in Bezug zu dem thermischen Stabilisierungsprozess als Vergleich (Erhitzung 30 min bei 60°C) dargestellt. Diese Ergebnisse zeigen, dass bei einer insgesamt hohen Energiezufuhr, z.B. einer Produkteingangstemperatur für die PEF-2 von 55 °C, einem spezifischen Energieeintrag von 55,7 kJ/kg sowie einer Produkteingangstemperatur für die PEF-2 von 60 °C und einem spezifischen Energieeintrag von 49,1 und 55 kJ/kg hohe Gehalte des Präparats an der lipophilen Verbindung erhalten werden, entsprechend keine anschließende Heißhaltung erforderlich ist oder auch den Gehalt verringern kann.

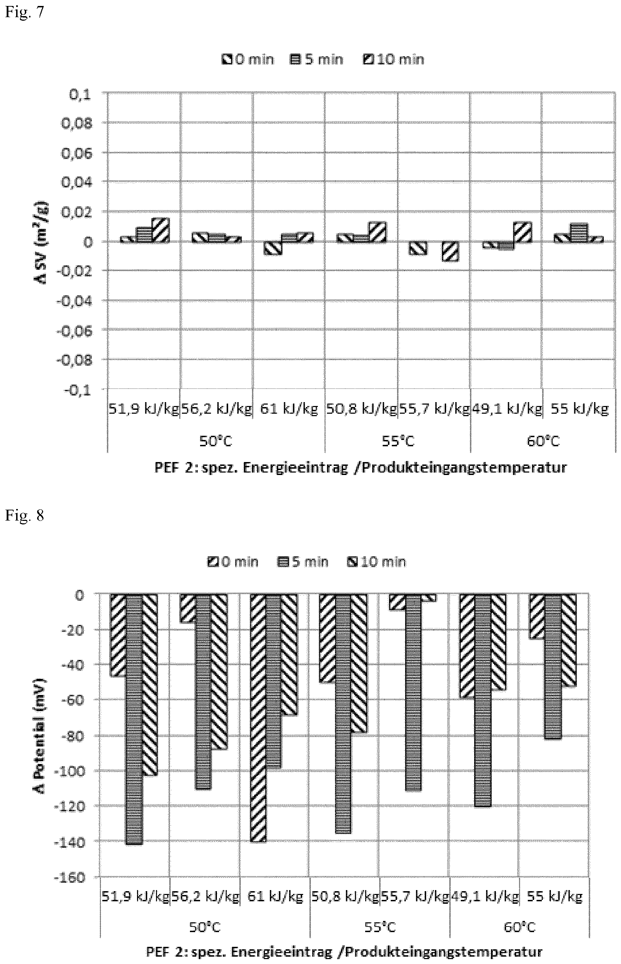

Die spezifische Oberfläche der Partikel des Präparats, in Figur 7 dargestellt, kann durch die Verfahrensbedingungen erhöht oder verringert werden. Auch bei diesem Präparat wird die Erhöhung der spezifischen Oberfläche auf den Zerfall von Aggregaten aus Caseinmicellen zurückgeführt, die Verringerung auf die Bildung von Aggregaten.

Das relative Oberflächenpotential der Präparate aus rehydratisierten Caseinmicellen ist in Figur 8 in Bezug zum Zustand vor der PEF 2-Behandlung dargestellt und zeigt, dass das Oberflächenpotential durch das Verfahren ohne oder mit Heißhaltung im Anschluss an die PEF-2 ein stärkeres negatives Oberflächenpotential ergibt. Von diesem stärkeren negativen Oberflächenpotential wird angenommen, dass es das Präparat bzw. die darin enthaltenen Partikel aus Caseinmicellen stabilisiert.

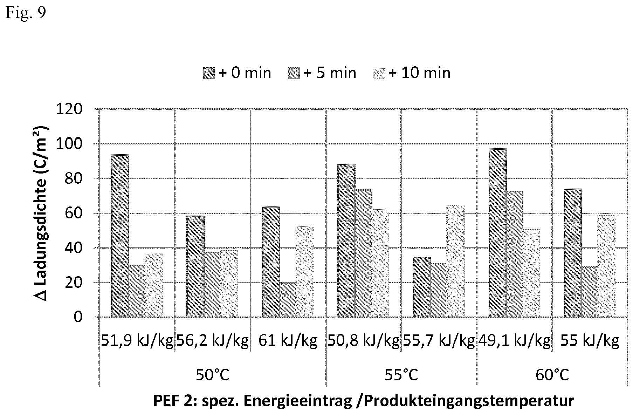

Die in Figur 9 gezeigten Messwerte für Ladungsdichten der Präparate aus rehydratisierten Caseinmicellen zeigen, dass die geringen spezifischen Oberflächen mit hohen Ladungsdichten verbunden sind.

Diese Ergebnisse könnten auch zeigen, dass die PEF-2 bei höherem Energieeintrag zur irreversiblen Bildung von Klumpen aus Caseinmicellen führt.

## Patentansprüche

1. Verfahren zur Herstellung eines wassermischbaren Präparats zumindest einer lipophilen Verbindung mit den Schritten
- Behandeln einer Suspension von Caseinmicellen in wässrigem Medium mit einem ersten gepulsten elektrischen Feld (PEF-1) mit einem spezifischen Energieeintrag von 20 bis 150 kJ/kg der wässrigen Suspension, einer elektrischen Feldstärke von 10 bis 20 kV/cm und einer Pulsdauer von 15 bis 25 µs, wobei die wässrige Suspension bevorzugt eine Temperatur von 1 bis 60 °C aufweist, zur Herstellung einer Suspension erster Caseinmicellen,
- Mischen der Suspension erster Caseinmicellen mit einer Lösung der lipophilen Verbindung zur Herstellung einer Mischung der Suspension erster Caseinmicellen mit der lipophilen Verbindung,
- Behandeln der Mischung der Suspension erster Caseinmicellen mit der lipophilen Verbindung mit einem zweiten gepulsten elektrischen Feld (PEF-2) mit einem spezifischen Energieeintrag von 40 bis 61 kJ/kg der Mischung zur Herstellung des Präparats.

2. Verfahren nach Anspruch 1 **dadurch gekennzeichnet, dass** das erste gepulste elektrische Feld einen spezifischen Energieeintrag von 35 kJ/kg der wässrigen Suspension, eine elektrischen Feldstärke von 11,5 kV/cm und eine Pulsdauer von 15 µs aufweist.

3. Verfahren nach einem der voranstehenden Ansprüche, **dadurch gekennzeichnet, dass** die Suspension von Caseinmicellen in wässrigem Medium mit dem ersten gepulsten elektrischen Feld bei einer Pulsfrequenz von 150 bis 250 Hz behandelt wird

4. Verfahren nach einem der voranstehenden Ansprüche, **dadurch gekennzeichnet, dass** das Lösungsmittel für die lipophile Verbindung mit dem wässrigen Medium mischbar ist.

5. Verfahren nach einem der voranstehenden Ansprüche, **dadurch gekennzeichnet, dass** die Suspension erster Caseinmicellen mit der lipophilen Verbindung vor der Behandlung mit dem zweiten gepulsten elektrischen Feld eine Temperatur von 35 bis 60 °C aufweist.

6. Verfahren nach einem der voranstehenden Ansprüche, **dadurch gekennzeichnet, dass** das zweite gepulste elektrische Feld eine elektrische Feldstärke von 10 bis 20 kV/cm, bevorzugt 11,5 kV/cm, eine Pulsdauer von 15 bis 25 µs, bevorzugt 15 µs, bei einer Pulsfrequenz von 150 bis 250 Hz aufweist.

7. Verfahren nach einem der voranstehenden Ansprüche, **dadurch gekennzeichnet, dass** die Suspension von Caseinmicellen in wässrigem Medium die Caseinmicellen in einer Konzentration von 2 bis 10 g/100g der Suspension enthält.

8. Verfahren nach einem der voranstehenden Ansprüche, **dadurch gekennzeichnet, dass** die Suspension von Caseinmicellen in wässrigem Medium einen pH-Wert von 6,2 bis 6,4 aufweist.

9. Verfahren nach einem der voranstehenden Ansprüche, **dadurch gekennzeichnet, dass** die Suspension von Caseinmicellen in wässrigem Medium eine Leitfähigkeit von 0,8 bis 1,5 mS/cm aufweist.

10. Verfahren nach einem der voranstehenden Ansprüche, **dadurch gekennzeichnet, dass** die Suspension von Caseinmicellen in wässrigem Medium vor dem Behandeln mit dem ersten gepulsten elektrischen Feld auf eine Temperatur von 2,5 bis 3,5 °C temperiert wird.

11. Verfahren nach einem der voranstehenden Ansprüche, **gekennzeichnet durch** das an die Behandlung mit dem zweiten gepulsten elektrischen Feld anschließende Heißhalten bei 55 bis 65 °C für bis zu 20 min.

12. Verfahren nach einem der voranstehenden Ansprüche, **dadurch gekennzeichnet, dass** das Präparat von der flüssigen Phase abgetrennt wird

13. Verfahren nach einem der voranstehenden Ansprüche, **dadurch gekennzeichnet, dass** das Präparat getrocknet wird.

14. Verfahren nach einem der voranstehenden Ansprüche, **dadurch gekennzeichnet, dass** kein Fett und kein Emulgator oder Tensid zugesetzt wird.

15. Verfahren nach einem der voranstehenden Ansprüche, **dadurch gekennzeichnet, dass** das Präparat in ein Lebensmittel eingemischt wird, dessen Fettgehalt maximal 2 Gew.-% beträgt.

## Claims

1. Process for producing a water-miscible preparation of at least one lipophilic compound with the steps of
- treating a suspension of casein micelles in an aqueous medium with a first pulsed electric field (PEF-1) having a specific energy input of 20 to 150 kJ/kg of the aqueous suspension, having an electric field strength of 10 to 20 kV/cm and having a pulse duration of 15 to 25 µs, wherein the aqueous suspension preferably has a temperature of 1 to 60 °C, for the production of a suspension of first casein micelles,
- mixing the suspension of first casein micelles with a solution of the lipophilic compound for the production of a mixture of the suspension of first casein micelles with the lipophilic compound,
- treating the mixture of the suspension of first casein micelles with the lipophilic compound with a second pulsed electric field (PEF-2) having a specific energy input of 40 to 61 kJ/kg of the mixture for the production of the preparation.

2. Process according to claim 1, **characterized in that** the first pulsed electric field has a specific energy input of 35 kJ/kg of the aqueous suspension, an electric field strength of 11.5 kV/cm and a pulse duration of 15 µs.

3. Process according to one of the preceding claims, **characterized in that** the suspension of casein micelles in aqueous medium is treated with the first pulsed electric field at a pulse frequency of 150 to 250 Hz.

4. Process according to one of the preceding claims, **characterized in that** the solvent for the lipophilic compound is miscible with the aqueous medium.

5. Process according to one of the preceding claims, **characterized in that** prior to the treatment with the second pulsed electric field the suspension of first casein micelles with the lipophilic compound has a temperature of 35 to 60 °C.

6. Process according to one of the preceding claims, **characterized in that** the second pulsed electric field has an electric field strength of 10 to 20 kV/cm, preferably 11.5 kV/cm, a pulse duration of 15 to 25 µs, preferably 15 µs, at a pulse frequency of 150 to 250 Hz.

7. Process according to one of the preceding claims, **characterized in that** the suspension of casein micelles in aqueous medium contains the casein micelles at a concentration of 2 to 10 g/100g of the suspension.

8. Process according to one of the preceding claims, **characterized in that** the suspension of casein micelles in aqueous medium has a pH of 6.2 to 6.4.

9. Process according to one of the preceding claims, **characterized in that** the suspension of casein micelles in aqueous medium has a conductivity of 0.8 to 1.5 mS/cm.

10. Process according to one of the preceding claims, **characterized in that** prior to the treatment with the first pulsed electric field the suspension of casein micelles in aqueous medium is temperatue-controlled to a temperature of 2.5 to 3.5 °C.

11. Process according to one of the preceding claims, **characterized by** hot-keeping at 55 to 65 °C for up to 20 min subsequent to the treatment with the second pulsed electric field.

12. Process according to one of the preceding claims, **characterized in that** the preparation is separated from the liquid phase.

13. Process according to one of the preceding claims, **characterized in that** the preparation is dried.

14. Process according to one of the preceding claims, **characterized in that** no fat and no emulsifier or surfactant is added.

15. Process according to one of the preceding claims, **characterized in that** the preparation is mixed into a foodstuff, the fat content of which is at maximum 2 wt.-%.

## Revendications

1. Procédé pour la préparation d'une préparation miscible à l'eau d'au moins un composé lipophile, comprenant les étapes suivantes
- traiter une suspension de micelles de caséine en milieu aqueux avec un premier champ électrique pulsé (PEF-1) ayant un apport d'énergie spécifique de 20 à 150 kJ/kg de la suspension aqueuse, une intensité de champ électrique de 10 à 20 kV/cm et une durée d'impulsion de 15 à 25 µs, la suspension aqueuse ayant de préférence une température de 1 à 60 °C, pour produire une suspension de premières micelles de caséine,
- mélanger la suspension de premières micelles de caséine avec une solution du composé lipophile pour produire un mélange de la suspension de premières micelles de caséine avec le composé lipophile,
- traiter le mélange de la suspension de premières micelles de caséine avec le composé lipophile avec un second champ électrique pulsé (PEF-2) ayant un apport d'énergie spécifique de 40 à 61 kJ/kg du mélange pour produire la préparation.

2. Procédé selon la revendication 1, **caractérisé en ce que** le premier champ électrique pulsé a un apport d'énergie spécifique de 35 kJ/kg de la suspension aqueuse, une intensité de champ électrique de 11,5 kV/cm et une durée d'impulsion de 15 µs.

3. Procédé selon l'une des revendications précédentes, **caractérisé en ce que** la suspension de micelles de caséine en milieu aqueux est traitée par le premier champ électrique pulsé à une fréquence d'impulsion de 150 à 250 Hz.

4. Procédé selon l'une des revendications précédentes, **caractérisé en ce que** le solvant du composé lipophile est miscible au milieu aqueux.

5. Procédé selon l'une des revendications précédentes, **caractérisé en ce que** la suspension de premières micelles de caséine avec le composé lipophile a une température de 35 à 60 °C avant le traitement par le second champ électrique pulsé.

6. Procédé selon l'une des revendications précédentes, **caractérisé en ce que** le second champ électrique pulsé a une intensité de champ électrique de 10 à 20 kV/cm, de préférence 11,5 kV/cm, une durée d'impulsion de 15 à 25 µs, de préférence 15 µs, à une fréquence d'impulsion de 150 à 250 Hz.

7. Procédé selon l'une des revendications précédentes, **caractérisé en ce que** la suspension de micelles de caséine en milieu aqueux contient les micelles de caséine à une concentration de 2 à 10 g/100g de la suspension.

8. Procédé selon l'une des revendications précédentes, **caractérisé en ce que** la suspension de micelles de caséine en milieu aqueux a un pH de 6,2 à 6,4.

9. Procédé selon l'une des revendications précédentes, **caractérisé en ce que** la suspension de micelles de caséine en milieu aqueux présente une conductivité de 0,8 à 1,5 mS/cm.

10. Procédé selon l'une des revendications précédentes, **caractérisé en ce que** la suspension de micelles de caséine en milieu aqueux est tempérée à une température de 2,5 à 3,5 °C avant le traitement par le premier champ électrique pulsé.

11. Procédé selon l'une des revendications précédentes, **caractérisé par** le maintien à chaud à 55 à 65 °C pendant une durée allant jusqu'à 20 minutes après le traitement avec le second champ électrique pulsé.

12. Procédé selon l'une des revendications précédentes, **caractérisé en ce que** la préparation est séparée de la phase liquide.

13. Procédé selon l'une des revendications précédentes, **caractérisé en ce que** la préparation est séchée.

14. Procédé selon l'une des revendications précédentes, **caractérisé en ce qu'**on n'ajoute pas de matière grasse ni d'émulsifiant ou de tensioactif.

15. Procédé selon l'une des revendications précédentes, **caractérisé en ce que** la préparation est mélangée à une denrée alimentaire dont la teneur en matières grasses est d'au plus 2 % en poids.
